# EUROPEAN PATENT APPLICATION

(11) **EP 4 729 077 A1**
(43) Date of publication of application: **22.04.2026**
(21) Application number: 25208141.9
(22) Date of filing: 10.10.2025
(51) Int. Cl.: A61L 2/104

(54) **UV-C STERILIZATION CONTROL**

(30) Priority: 18.10.2024 GB 202415331
(71) Applicant: Aspen Pumps Limited, Hailsham, East Sussex BN27 3WA (GB)
(72) Inventor: MARTIN, Walter, Glenwherry (IE); JONES, Ciaran, East Sussex, BN27 3WA (GB); CAPON, Phil, East Sussex, TN33 0HS (GB); COSTELLO, Ken, East Sussex, BN27 3WA (GB)
(74) Representative: HGF

(57) **Abstract**

There is provided a method of sterilizing an interior surface of a reservoir (10) in a pump system, comprising the steps of: sensing a level of liquid in the reservoir (10); and illuminating the surface with UV-C in dependence on the sensed liquid level being at or below a predetermined level at which the surface is at least partially exposed. Improved efficiency of decontamination is thus achieved by only activating the UV-C when the relevant surface is exposed. The UV-C illumination may be coordinated with operation of the pump so that the UV-C light source (60) is activated only when the pump (40) is not pumping and vice versa.

## Description

This invention relates to UV-C sterilization control, in particular to a method of sterilizing an interior surface of a reservoir in a pump system, such as a condensate reservoir of a refrigeration or air conditioning unit, and to an associated pump system.

### BACKGROUND

Condensate pump assemblies are used to pump liquid condensate from appliances that produce condensate, for example an air conditioning system, a condensing boiler system or a refrigerator, out of a room or building. In a typical air conditioning system, the air conditioning unit produces liquid condensate, i.e. water, which drains from the air conditioning unit to a liquid reservoir in a condensate pump assembly, often located near the air conditioning unit. After sustained operation of the condensate pump assembly, contaminants such as dirt may enter the reservoir and are not removed by the pump. As there are humid conditions within the reservoir of a condensate pump, care should be taken to avoid mould or other bacteriological growth, such as biofilm, within the reservoir which may result in harmful substances being introduced into the pump or products being refrigerated. Similar concerns apply with respect to condensate pumps of refrigeration units, particularly in industrial or commercial settings, which may also be installed in confined spaces. This can make cleaning of these units challenging and sometimes dangerous, particularly where chemicals are required to properly clean the units.

Accurately detecting the presence of liquids, in particular the accurate measurement of liquid levels, is important in a number of different situations. One such example is in the liquid reservoir of a condensate pump assembly, as described above, where it is important to accurately detect condensate levels to control operation of a pump to evacuate the condensate from the reservoir and to ensure that the level of condensate is not too high or too low. If the condensate level is too high, or overflowing in extreme cases, this can result in water damage to the surfaces or structures surrounding or supporting the air conditioning unit. If the condensate level is too low, this can result in air being drawn into the pump motor, which in turn causes significant noise to be generated during normal operation of the condensate pump. This is particularly undesirable when the air-conditioning units are installed in residential or commercial spaces in the presence of people. In manufacturing facilities, storage tanks or containers can contain liquids that need to be kept above a threshold to avoid equipment becoming damaged or production stopping completely. Often such containers are monitored remotely and therefore it is important to know that when the liquid level within such a container drops below a certain level, the sensors measuring the liquid level are able to accurately and reliably detect this.

One way to detect liquid levels within a reservoir is to use a capacitance sensor. For example, it is known to incorporate a capacitance sensor within a reservoir in a condensate pump of an air-conditioning system, so that the condensate pump can be operated based on the measured condensate level. Securing the capacitance sensor within the housing of the condensate pump and having a portion of the housing extend into the reservoir volume enables the sensing element of the capacitance sensor to be in close proximity to the condensate. This allows the sensor to detect the liquid level without needing to be in direct contact with the condensate itself.

Whilst such non-contact sensors are desirable, one of the most significant issues that impedes the reliability of capacitive water sensing methods is the formation of bacteriological deposits on the sensor. These are an enabler for contamination of the sensor and eventually cause the capacitance value measured by the sensor to change significantly, leading to erroneous readings being outputted and thus rendering the sensor inoperable. Normally, regular preventative maintenance needs to be carried out, to clean these deposits. This can be expensive and requires site visits and disassembly of the product. Attempts have been made to introduce biocidal chemicals into the pump reservoir to solve the problem, but these have two very serious disadvantages. Firstly, the biocidal products change the dielectric constant of the water and also affect the capacitance value being read; and secondly biocidal products are generally being phased out due to their negative environmental impacts.

A similar effect occurs with float-based water sensing where contamination can cause the float to 'stick' and no longer or inaccurately respond to water level changes.

The present invention seeks to address at least some of these problems.

### BRIEF SUMMARY OF THE DISCLOSURE

In accordance with a first aspect of the present invention there is provided a method of sterilizing an interior surface of a reservoir in a pump system, comprising the steps of: sensing a level of liquid in the reservoir; and illuminating the surface with UV-C in dependence on the sensed liquid level being at or below a predetermined level at which the surface is at least partially exposed.

Advantageously, the illumination only occurs when the surface is at least partially, but preferably substantially wholly, free from liquid to achieve maximum benefit as any media between the surface and the illumination source will reduce its effectiveness.

The method may further comprise a step of pumping liquid out of the reservoir in dependence on the sensed liquid level being above said predetermined level. Accordingly, illumination of the UV-C and the pumping may be coordinated such that the illumination only occurs once pumping has stopped, and *vice versa.* This may be beneficial for keeping the power requirements for the system to a minimum and may also help to keep the size of the system down.

The steps of sensing and illuminating may be controlled by a microprocessor. Thus, the microprocessor may be in operative communication with a UV-C light source to control said illuminating, and may be in operative communication with a liquid level sensor for receiving a trigger signal therefrom indicative of the sensed liquid level being below the predetermined level, whereby the microprocessor can generate an illumination control signal to activate the UV-C illumination in response to receiving said trigger signal. In certain embodiments, the microprocessor may be adapted to control the intensity of illumination. For example, the intensity may be dependent on a sensed level of contamination on the illuminated surface. Where the UV-C light source comprises an LED, the intensity of illumination may be controlled by pulse width modulation.

Where the method includes a pumping step, the microprocessor may further be in operative communication with a pump to control said pumping. Accordingly, the microprocessor may generate a pump control signal to stop the pump from pumping in response to receiving said trigger signal. Such microprocessor control ensures that the pumping and illumination operations can be well coordinated, for example to ensure that the illumination only occurs once the pump has stopped pumping and *vice versa.*

The surface that is illuminated may comprise a sensing surface of a liquid level sensor device in the reservoir. The liquid level sensor device may be used to detect a level of liquid within the reservoir. Accordingly, the step of sensing a level of liquid in the reservoir may comprise sensing a level of liquid in the reservoir with said liquid level sensor. The liquid level sensor may comprise a capacitive sensor. Also, or instead, the liquid level sensor may comprise a float device. Additionally or instead, the surface that is illuminated may comprise a pick-up tube of the pump within the reservoir.

The method may further comprise a step of detecting the status of an interlock safety device. As such, the step of illuminating the surface with UV-C may be further in dependence on said status. Since exposure to UV-C light can be dangerous, such a safety device can be employed to ensure that illumination only occurs when it is known that it will be safely contained within the interior of the reservoir. For example, the interlock may be associated with the proper attachment of a cover to the reservoir.

According to a second aspect of the invention, there is provided a pump system comprising: a reservoir including an interior surface; a pump adapted to pump liquid out of the reservoir; a liquid level sensor adapted to detect a level of liquid in the reservoir; and a UV-C light source. The UV-C light source is adapted to illuminate said surface in response to receiving a signal from the liquid level sensor indicative of the sensed liquid level being at or below a predetermined level at which the surface is at least partially exposed.

The pump system may comprise a condensate pump and the liquid in the reservoir may thus comprise condensate water.

As noted above in respect of the associated method according to the first aspect, this is beneficial in that the illumination only occurs when the surface is at least partially, but preferably substantially wholly, free from liquid to achieve maximum benefit as any media between the surface and LED will reduce its effectiveness.

The pump system may further comprise a microprocessor, wherein the microprocessor is in operative communication with the UV-C light source, and wherein the microprocessor is in operative communication with the liquid level sensor and is adapted to receive a trigger signal therefrom indicative of the sensed liquid level being below the predetermined level, whereby the microprocessor is adapted to generate an illumination control signal to activate the UV-C light source in response to receiving said trigger signal.

The microprocessor may be further in operative communication with the pump and be adapted to generate a pump control signal to stop the pump from pumping in response to receiving said trigger signal.

The surface that is illuminated may comprise a sensing surface of the liquid level sensor. In some embodiments, the liquid level sensor may comprise a capacitive sensor. In some embodiments, the liquid level sensor may also or instead comprise a float device.

The pump may include a pick-up tube disposed in the reservoir, and said surface that is illuminated may comprise a surface of said pick-up tube.

The UV-C light source may comprise an LED. Preferably, the LED is located within the reservoir. Accordingly, the illumination can be contained within the interior of the reservoir, which is beneficial from a safety perspective. Moreover, there is no need then to provide a mechanism for the passage of the UV-C illumination into the inside of the reservoir, such as by having at least a portion of the reservoir constructed from UV-C transparent material.

More than one UV-C light source may be included. The or each UV-C light source may be directed to illuminate a particular portion of said interior surface. For example, several LEDs may each be directed towards the same surface to provide effective coverage. Optionally, several LEDs may each be directed towards different surfaces, e.g. one towards a sensing surface of a capacitive sensor and another towards a pick-up tube of the pump.

The pump system may further comprise an interlock safety device, wherein activation of the UV-C light source is further dependent on a status of the interlock. The pump system may further comprise a reservoir cover, and the status of the interlock safety device may be determinative of the cover being correctly attached to the reservoir to enclose the interior of the reservoir. Accordingly, activation of the UV-C light source can only occur when the cover is correctly attached.

At least the interior surface of the reservoir may be made of UV-stable materials.

### BRIEF DESCRIPTION OF THE DRAWINGS

Embodiments of the invention are further described hereinafter with reference to the accompanying drawings, in which:
Figure 1 is a schematic illustration of an example condensate pump incorporating a UV-C sterilization system according to the invention;
Figure 2 is a cross sectional side elevation view of a reservoir portion of a condensate pump incorporating a UV-C sterilization system according to the invention, with liquid at a high level in the reservoir;
Figure 3a is an end plan view of the pump of Fig. 2;
Figure 3b is a cross sectional side elevation view of the reservoir portion, corresponding to Fig. 2 and taken through section B-B of Fig. 3a, and with liquid at a low level;
Figure 4 is a first perspective view of the reservoir portion, with cover omitted; and
Figure 5 is a second perspective view of the reservoir portion of Fig. 3b from an opposed end to Fig. 4, with cover omitted and having a cutaway through the reservoir to show the UV-C sterilization system within.

### DETAILED DESCRIPTION

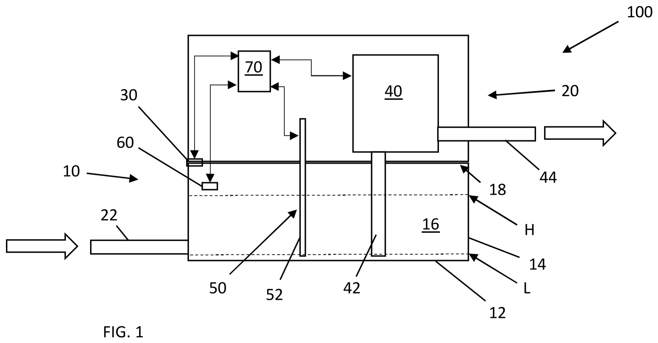

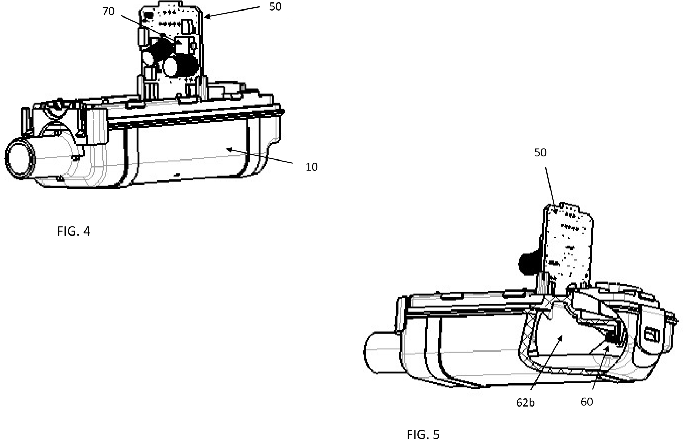

A reservoir portion 100 of a condensate pump system is illustrated by reference to Figures 1 to 5. The reservoir portion 100 comprises a reservoir 10 defined by a base wall 12 and sidewalls 14 circumscribing the base wall 12, together defining an interior volume 16. A cover 20 is coupled to an upper end of the reservoir 10, for example at a rim 18 formed at the upper ends of the sidewalls 14. An interlock safety device 30 is included at the interface of the cover 20 and the reservoir 10 for a purpose to be described below.

The reservoir 10 is in fluid communication with a condensate-producing unit (not shown) such as a refrigerator or an air-conditioner via an inlet 22. A pump 40 is in fluid communication with the interior volume 16 of the reservoir 10 via a pick-up tube 42, positioned to be able to pump condensate 80 from the reservoir down to a minimum fill level L. Condensate extracted from the reservoir by the pump 40 is ejected via outlet 44.

A liquid level sensor 50 is positioned in the reservoir portion 100 for detecting the level of condensate within the reservoir 10, which may be between the minimum, low level L and a maximum, high level H. As illustrated, the liquid level sensor 50 is in the form of a capacitive sensor, with a sensing surface 52. As known in the art, a liquid level signal is generated by the capacitance sensor 50 in dependence on the amount of the surface 52 that is covered by the liquid in the reservoir 10 - i.e. the height of the liquid in the interior volume 16.

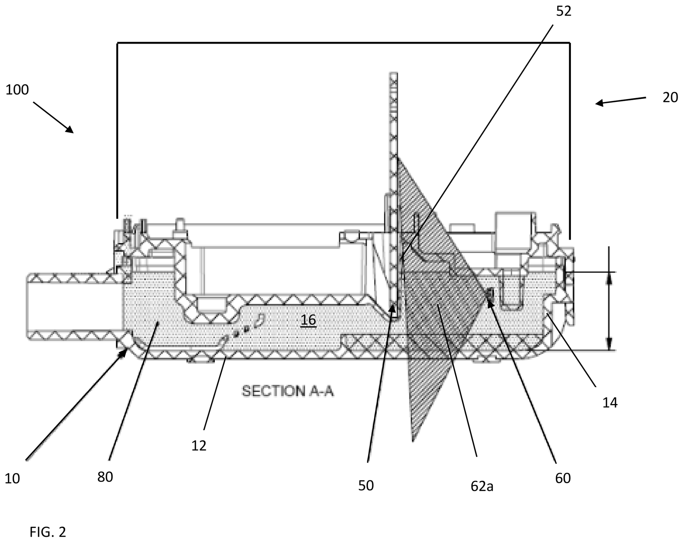

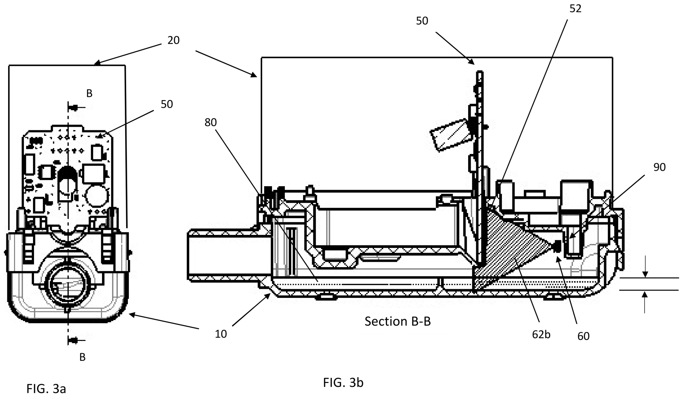

A UV-C light source 60, typically in the form of one or more UV-C LEDs, is positioned in the reservoir portion 100 for illuminating at least one interior surface of the reservoir 10. In one embodiment, the surface to be illuminated is the sensing surface 52 of the capacitance sensor 50. As illustrated, the UV-C light source 60 is positioned on an interior surface of the reservoir portion, such as a surface 90 substantially parallel to the sensing surface 52, such that the pattern 62a,62b of UV-C emissions emitted by the light source illuminate the sensing surface 52, but it will be understood that other arrangements are possible. In Fig. 2, the pattern 62a is relatively wide, and may thus have a relatively lower energy density than the pattern 62b of Fig. 3b. In another embodiment, the exterior surface of the pick-up tube 42 may also or instead be the illuminated surface. More generally, any interior surface within the interior volume 16 of the reservoir 10 may be illuminated, including the interior surfaces of the sidewalls 14. It is particularly the surfaces that come into contact with the liquid and which are thus particularly prone to bacteriological contamination that are to be targeted. As is known, UV-C light sources generate ultraviolet light at a frequency that can destroy the DNA of bacteria and viruses.

A single UV-C light source may be adapted to illuminate several different surfaces, for example through appropriate lensing or light pipe arrangements. Alternatively, different UV-C light sources may be positioned to illuminate different respective surfaces. For simplicity, henceforth reference to the UV-C light source 60 covers both a single such light source and plural such light sources.

The UV-C light source 60 is adapted to illuminate said surface in response to receiving a signal from the liquid level sensor 50 indicative of the sensed liquid level being at or below a predetermined level at which the surface is at least partially exposed. This may correspond to the liquid being at the minimum fill level L. Turning the UV-C light source 60 ON only when the associated surface is at least partially exposed achieves maximum benefit because any liquid between the surface and the illumination source will reduce its effectiveness at decontaminating that surface. Conversely, turning the UV-C light source 60 OFF when the associated surface is at least partially covered by the liquid saves energy. The decontamination through UV-C illumination is thus done in a most energy-efficient manner.

Control of the illumination may be automated. Accordingly, in preferred embodiments, the pump system further comprises a microprocessor 70, which is in operative communication with the UV-C light source 60 and with the liquid level sensor 50. The microprocessor 70 is adapted to receive a trigger signal from the liquid level sensor 50 indicative of the sensed liquid level being below the predetermined level - e.g. the low level L. On receipt of the trigger signal, the microprocessor 70 generates an illumination control signal to activate (turn ON) the UV-C light source 60. Conversely, on detecting that the sensed liquid level has risen above the predetermined level, the liquid level sensor 50 may send a stop signal to the microprocessor 70 (or may stop sending the trigger signal), at which point the microprocessor 70 generates an illumination control signal to deactivate (turn OFF) the UV-C light source 60.

In certain embodiments, the intensity of the UV-C LED(s) 60 can be controlled via PWM (pulse with modulating) the power to the LED(s). In this manner, surfaces that are determined to be less contaminated can receive a lower exposure with consequent savings in power.

The microprocessor 70 is further in operative communication with the pump 40 for controlling operation thereof, for example dependent on the liquid level as sensed by the liquid level sensor 50. Accordingly, on receipt of the trigger signal, the microprocessor 70 also generates a pump control signal to stop the pump from pumping (i.e. pump OFF). Conversely, on detecting that the sensed liquid level has risen above the predetermined level, the liquid level sensor 50 may send a signal to the microprocessor 70 (or may stop sending the trigger signal), at which point the microprocessor 70 generates a pump control signal to activate (turn ON) the pump 40.

As such, the operations of the pump 40 on the one hand and the UV-C light source 60 on the other hand may be coordinated via the microcontroller 70, controlling the intensity and illumination of the UV-C light source 60, in conjunction with the pumping cycle. This coordination ensures that the energy draw is kept to a minimum, which in turn means that the size and cost of the associated componentry can be minimised. This is important as the proportion of power required by LEDs relative to that required for pumping is significant, resulting in increased physical size of the pump.

Where an interlock safety device 30 is included, it may be in operative communication with the microprocessor 70 so that activation of the UV-C light source is further dependent on a status of the interlock. The safety interlock device 30 may generate an interlock signal indicative, for example, of the reservoir cover 20 being correctly attached to the reservoir 10. The microprocessor 70 may be adapted so that it is unable to generate the illumination control signal to activate (turn ON) the UV-C light source 60 unless it receives the interlock signal. Accordingly, activation of the UV-C light 60 source can only occur when the cover 20 is correctly attached, thereby ensuring that UV-C light does not escape from the enclosed interior.

At least the interior surface of the reservoir 10 is made of UV-stable materials so as not to be affected by the exposure to the UV-C illumination.

In summary, there is provided a method of sterilizing an interior surface of a reservoir 10 in a pump system, comprising the steps of: sensing a level of liquid in the reservoir 10; and illuminating the surface with UV-C in dependence on the sensed liquid level being at or below a predetermined level at which the surface is at least partially exposed. Improved efficiency of decontamination is thus achieved by only activating the UV-C when the relevant surface is exposed. The UV-C illumination may be coordinated with operation of the pump so that the UV-C light source 60 is activated only when the pump 40 is not pumping and vice versa.

Although described primarily by reference to the liquid level sensor in the form of a capacitive sensor, it is to be understood that certain advantages of the invention also apply where the liquid level sensor takes different forms, such as a float sensor. In such embodiments, the surfaces of the float sensor that are prone to 'sticking' due to bacteriological deposits may be targeted by the UV-C illumination.

Also, although the UV-C light source has primarily been described in terms of embodiments where it comprises a UV-C LED, which is advantageous for microprocessor control due to its ability to rapidly turn ON and OFF in succession, to facilitate PWM modulation, for example, it will be understood that alternative forms of UV-C light source could instead be used and provide the benefit of improved efficiency of decontamination by only activating when the relevant surface is exposed.

Further, whereas the light source 60 has been described as located within the reservoir portion 100, it could in some embodiments be positioned externally provided that a path to the relevant internal surface is provided, for example by an opening or a UV-C transmissive window. However, locating the light source 60 internally of a light-tight enclosure formed of UV-C blocking materials is beneficial from a safety perspective to minimise the risk of UV-C rays from escaping.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. A method of sterilizing an interior surface of a reservoir in a pump system, comprising the steps of:
sensing a level of liquid in the reservoir; and
illuminating the surface with UV-C in dependence on the sensed liquid level being at or below a predetermined level at which the surface is at least partially exposed.

2. The method of claim 1, further comprising a step of pumping liquid out of the reservoir in dependence on the sensed liquid level being above said predetermined level.

3. The method of claim 1 or claim 2, wherein said steps of sensing and illuminating are controlled by a microprocessor, wherein the microprocessor is in operative communication with a UV-C light source to control said illuminating, and wherein the microprocessor is in operative communication with a liquid level sensor and receives a trigger signal therefrom indicative of the sensed liquid level being below the predetermined level, whereby the microprocessor generates an illumination control signal to activate the UV-C illumination in response to receiving said trigger signal.

4. The method of claim 3, when dependent on claim 2, wherein the microprocessor is in operative communication with a pump to control said pumping, whereby the microprocessor generates a pump control signal to stop the pump from pumping in response to receiving said trigger signal.

5. The method of any preceding claim, wherein said surface comprises a sensing surface of a liquid level sensor device in the reservoir, optionally wherein said step of sensing a level of liquid in the reservoir comprises sensing a level of liquid in the reservoir with said liquid level sensor, optionally wherein liquid level sensor comprises a capacitive sensor device.

6. The method of any preceding claim, further comprising a step of detecting the status of an interlock safety device, and wherein the step of illuminating the surface with UV-C is further in dependence on said status.

7. A pump system comprising:
a reservoir including an interior surface;
a pump adapted to pump liquid out of the reservoir;
a liquid level sensor adapted to detect a level of liquid in the reservoir; and
a UV-C light source;
wherein the UV-C light source is adapted to illuminate said surface in response to receiving a signal from the liquid level sensor indicative of the sensed liquid level being at or below a predetermined level at which the surface is at least partially exposed.

8. The pump system of claim 7, further comprising a microprocessor, wherein the microprocessor is in operative communication with the UV-C light source, and wherein the microprocessor is in operative communication with the liquid level sensor and is adapted to receive a trigger signal therefrom indicative of the sensed liquid level being below the predetermined level, whereby the microprocessor is adapted to generate an illumination control signal to activate the UV-C light source in response to receiving said trigger signal.

9. The pump system of claim 7, wherein the microprocessor is further in operative communication with the pump and is adapted to generate a pump control signal to stop the pump from pumping in response to receiving said trigger signal.

10. The pump system of any of claims 7 to 9, wherein said surface comprises a sensing surface of said liquid level sensor.

11. The pump system of any of claims 7 to 10, wherein said liquid level sensor comprises a capacitive sensor.

12. The pump system of any of claims 7 to 11, wherein said pump includes a pick-up tube disposed in the reservoir, and said surface comprises a surface of said pick-up tube.

13. The pump system of any of claims 7 to 12, wherein the UV-C light source comprises an LED.

14. The pump system of any of claims 7 to 13, further comprising an interlock safety device, wherein activation of the UV-C light source is further dependent on a status of the interlock, optionally further comprising a reservoir cover, wherein the status of the interlock safety device is determinative of the cover being correctly attached to the reservoir to enclose the interior of the reservoir, whereby activation of the UV-C light source can only occur when the cover is correctly attached.

15. The pump system of any of claims 7 to 14, wherein at least the interior surface of the reservoir is made of UV-stable materials.
